Europäisches Patentamt
European Patent Office
Office européen des brevets

(19)

(11) Numéro de publication: **0 344 816**
**A1**

(12)
# DEMANDE DE BREVET EUROPEEN

(21) Numéro de dépôt: **89112013.1**

(22) Date de dépôt: **29.01.87**

(51) Int. Cl.4: **G01N 33/534 , //C07C121/75, C07B59/00,C07D233/68**

(30) Priorité: **29.01.86 FR 8601220**

(43) Date de publication de la demande: **06.12.89 Bulletin 89/49**

(60) Numéro de publication de la demande initiale en application de l'article 76 CBE : **0 235 000**

(84) Etats contractants désignés: **CH DE FR GB IT LI NL**

(71) Demandeur: **ROUSSEL-UCLAF**
**35, boulevard des Invalides**
**F-75007 Paris(FR)**

(72) Inventeur: **Demoute, Jean-Pierre**
**65, Avenue Foch**
**F-93360 Neuilly Plaisance(FR)**
Inventeur: **Touyer, Gaetan**
**143, Avenue Jean Jaurès**
**F-75019 Paris(FR)**
Inventeur: **Mouren, Michel**
**15, rue de Mirbel**
**F-75005 Paris(FR)**

(74) Mandataire: **Tonnellier, Marie-José et al**
**111, route de Noisy B.P. no 9**
**F-93230 Romainville(FR)**

(54) **Pyréthrinoides marqués à l'iode, leur procédé et intermédiaires de préparation et leur application aux dosages radioimmunologiques.**

(57) Application des produits de formule

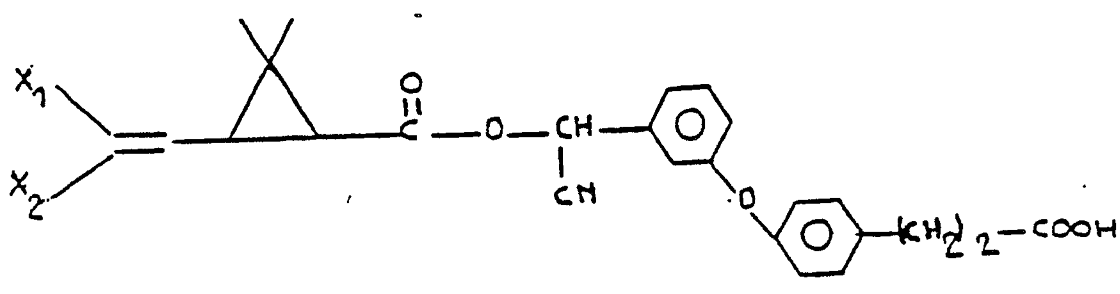

ou une de leurs formes activés,
dans laquelle $X_1$ représente un atome d'halogène ou un radical trifluorométhyle, $X_2$ représente un atome d'halogène à la préparation d'antigènes par couplage à une protéine immunogène.
Les antigènes ainsi obtenus trouvent une application dans les dosages radioimmunologiques.

EP 0 344 816 A1

**Pyréthrinoïdes marqués à l'iode, leur procédé et intermédiaires de de préparation et leur application aux dosages radioimmunologiques.**

La présente invention a pour objet de nouveaux dérivés pyréthrinoïdes radioactifs marqués à l'iode, leur procédé et leurs intermédiaires de préparation et leur application aux dosages radioimmunologiques.

Plus particulièrement, elle a pour objet des dérivés de formule générale (I) :

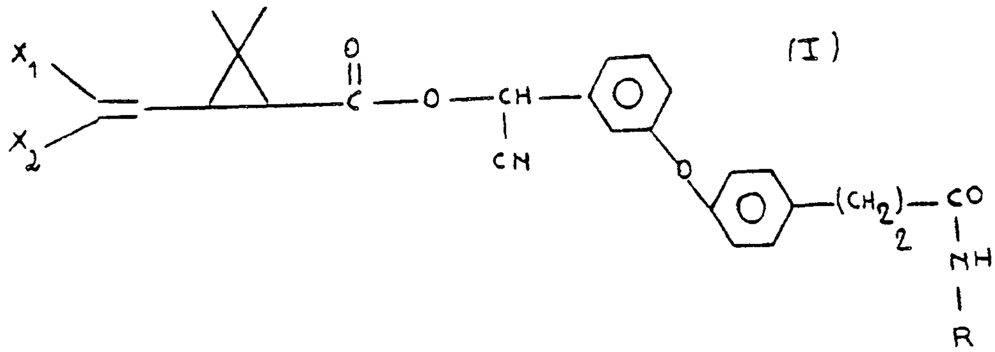

(I)

dans laquelle $X_1$ représente un atome d'halogène ou un radical trifluorométhyle, $X_2$ représente un atome d'halogène et R représente le reste d'un acide aminé $R-NH_2$ ou le reste d'un dérivé de ce dernier, ce reste possédant un groupe accepteur d'iode et étant marqué à l'iode $^{125}$ ou $^{131}$, ces dérivés se présentant sous l'une quelconque de leurs formes isomères ou sous forme de mélanges d'isomères.

$X_2$ et $X_1$ lorsqu'ils représentent un atome d'halogène, peuvent être un atome de fluor, de chlore, de brome ou d'iode.

Par mélange d'isomères, on entend tous les mélanges d'isomères possibles, y compris les racémates.

L'invention a plus particulièrement pour objet les dérivés radioactifs marqués à l'iode de formule générale (I) dans laquelle R représente le reste d'un acide aminé $RNH_2$ ou le reste d'un dérivé de ce dernier choisi dans le groupe constitué par l'histidine, la tyrosine, l'histidinol, l'histamine, la tyramine et le tyrosinate de méthyle marqués, à l'iode $^{125}$ ou $^{131}$.

L'invention a notamment pour objet les dérivés de formule (I) dans laquelle $X_1$ représente un atome de chlore ou de brome ou un radical trifluorométhyle et $X_2$ représente un atome de chlore ou de brome.

L'invention a plus particulièrement pour objet les dérivés de formule (I) dans laquelle $X_1$ et $X_2$ représentent un atome de brome et tout particulièrement :

le /1R-/1 alpha S, 2 alpha /4-/3-/cyano///3-(2,2-dibromoéthényl) 2,2-diméthyl cyclopropyl/carbonyl/oxy/méthyl/phénoxy/benzène-N-/2-(2-iodo $^{125}$I 4-1H-imidazolyl) éthyl/propanamide de formule :

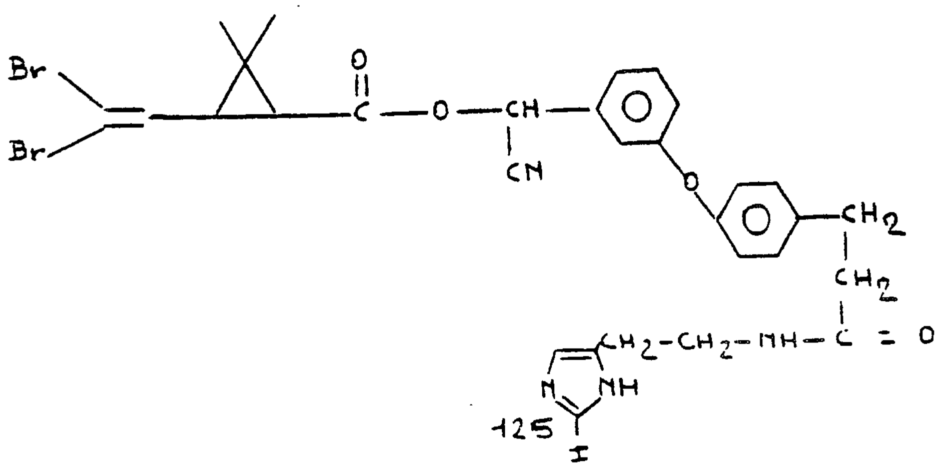

L'invention a également pour objet un procédé de préparation des produits répondant à la formule générale (I) ci-dessus.

Ce procédé est caractérisé en ce que l'on fait réagir un produit de formule (II) :

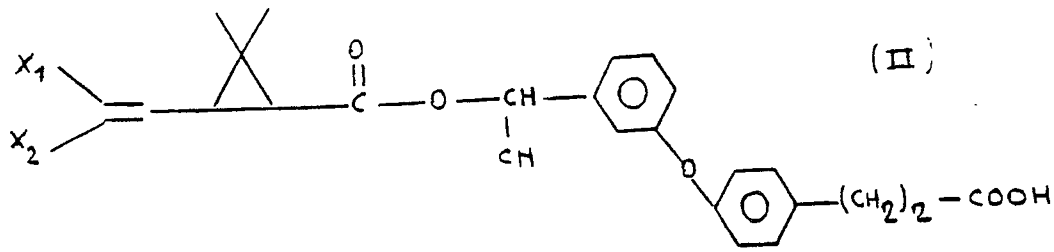

(II)

dans laquelle $X_1$ et $X_2$ ont la signification précitée, ce produit se présentant sous l'une quelconque de ses formes isomères ou sous forme de mélanges d'isomères, avec un réactif fixateur d'un groupement activateur de la fonction carbonyle pour obtenir le produit de formule (III) :

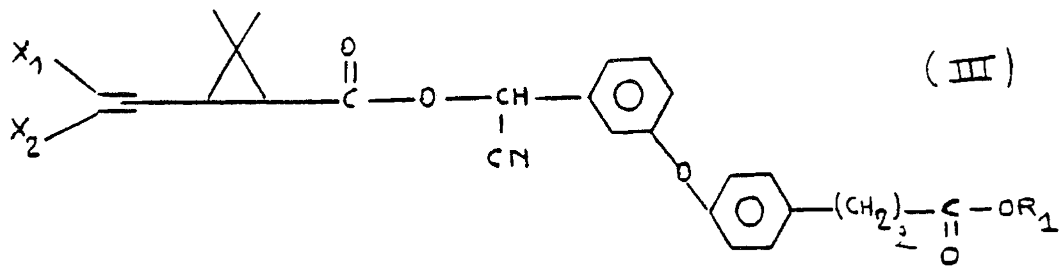

(III)

dans laquelle $X_1$ et $X_2$ ont la signification précitée et $R_1$ représente un groupement activateur de la fonction carbonyle que l'on fait réagir avec un acide aminé $RNH_2$ possédant un groupe accepteur d'iode marqué à l'iode $^{125}$ ou $^{131}$ ou avec un dérivé de cet acide aminé et obtient le produit cherché de formule générale (I), que l'on purifie par des moyens physiques.

Dans des conditions préférentielles de mise en oeuvre du procédé :

- l'acide aminé accepteur d'iode ou le dérivé d'acide aminé est choisi dans le groupe constitué par l'histidine, la tyrosine, l'histidinol, l'histamine, la tyramine et le tyrosinate de méthyle ;
- on fixe sur la fonction acide un groupement activateur de la fonction carbonyle en faisant agir un halogénoformiate d'alcoyle dans lequel le radical alcoyle renferme au plus 6 atomes de carbone et on opère en présence d'une base tertiaire, en milieu anhydre et sous atmosphère inerte.

Dans des conditions préférentielles de mise en oeuvre, le procédé est caractérisé en ce que l'halogénoformiate d'alcoyle est le chloroformiate d'isobutyle et on opère en présence de tri-n-butyl amine.

L'invention a notamment pour objet un procédé de préparation des composés de formule générale (I) caractérisé en ce que l'acide aminé ou son dérivé que l'on fait réagir avec le produit de formule générale (III) dans laquelle $R_1$ représente un groupement activateur de la fonction carbonyle, est l'histamine marquée à l'iode $^{125}$ ou $^{131}$ ou un dérivé et on opère sous atmosphère inerte.

L'invention a plus précisément pour objet un procédé de préparation tel que défini précédemment, caractérisé en ce que l'on obtient le produit de formule :

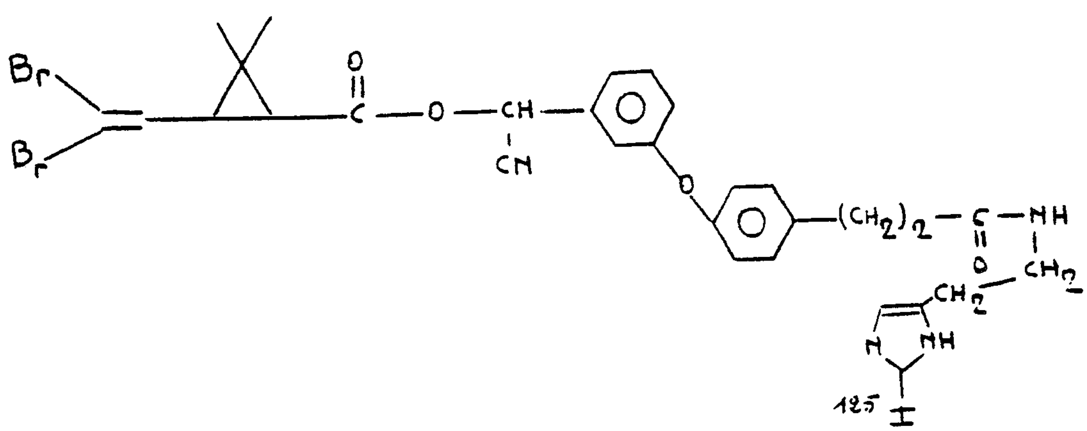

L'invention a également pour objet l'application des produits de formule générale (I) à l'étude et au dosage radioimmunologique des esters de l'alcool alpha-cyano 3-phénoxy benzylique correspondants (produit B) dans les fluides biologiques, chez l'homme ou l'animal.

L'invention a notamment pour objet l'application du /1R /1 alpha S, 2 alpha /4-/3-(cyano///3-(2,2-dibromo éthényl) 2,2-diméthyl cyclopropyl/carbonyl/ oxy/méthyl/phénoxy/benzène-N-/2-(2-iodo $^{125}$I 4-1 H imidazolyl)-éthyl/ propanamide (produit A) à l'étude et aux dosages radioimmunologiques de la deltamethrine (produit $B_1$) dans les fluides biologiques, chez l'homme ou l'animal.

L'invention a également pour objet, à titre de produits industriels nouveaux et notamment à titre de produits intermédiaires nécessaires à l'exécution du procédé de l'invention, les produits de formule (II) et (III) :

(II)

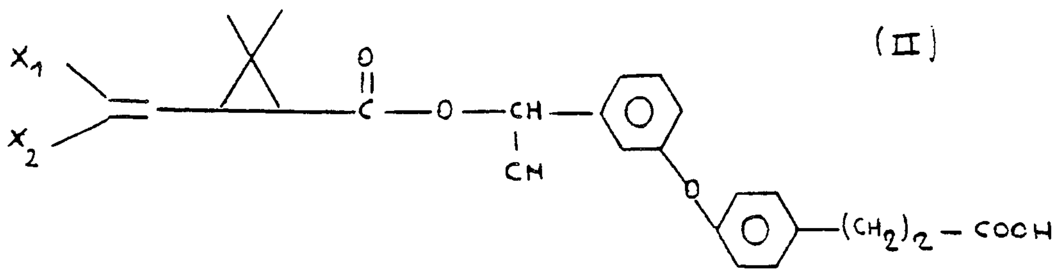

(III)

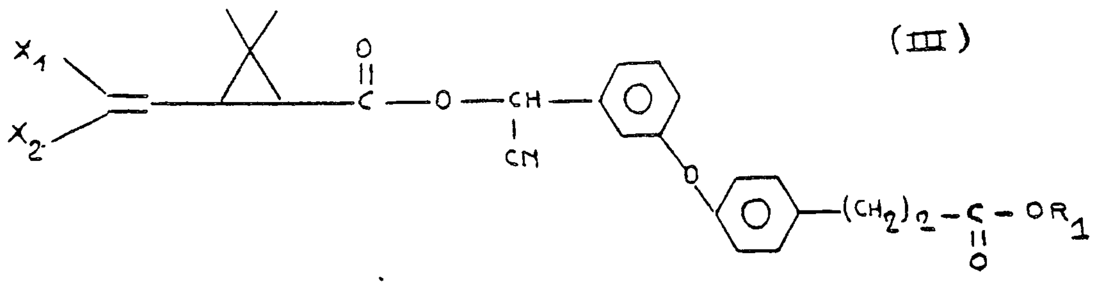

dans lesquelles $X_1$, $X_2$ et $R_1$ ont les significations précitées, sous l'une quelconque de leurs formes isomères et sous forme de mélanges d'isomères.

Parmi ces produits, l'invention a plus particulièrement pour objet le produit répondant à la formule III dans laquelle $R_1$ représente un radical - $\underset{\underset{O}{\|}}{C}$ -O-$CH_2CH(CH_3)_2$ et $X_1$ et $X_2$ représentent un atome de brome, à savoir le produit de formule :

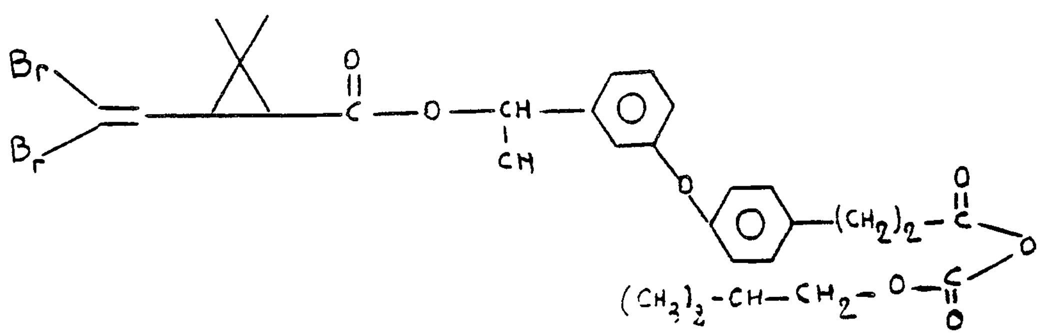

Les produits de formule (II) sont des produits de départ utiles également pour la préparation d'antigènes, ceux-ci étant eux-mêmes nécessaires aux dosages radioimmunologiques des produits B précités.

L'application des produits de formule (II) à la préparation d'antigènes fait l'objet également de la présente invention. Elle est caractérisée en ce que l'on conjugue les produits de formule (II) ou une forme activée avec de l'albumine sérique bovine (B.S.A.) ou avec une autre protéine immunogène telle que l'albumine sérique humaine, la thyroglobuline porcine ou bovine, l'anatoxine tétonique, l'anatoxine diphtérique, l'ovalbumine, l'hémocyanine de Patelle (KLH) ou les gamma globulines, et obtient les antigènes recherchés

Dans des conditions préférentielles de mise en oeuvre, cette application est caractérisée en ce que :
- l'on fait réagir un produit de formule (II) :

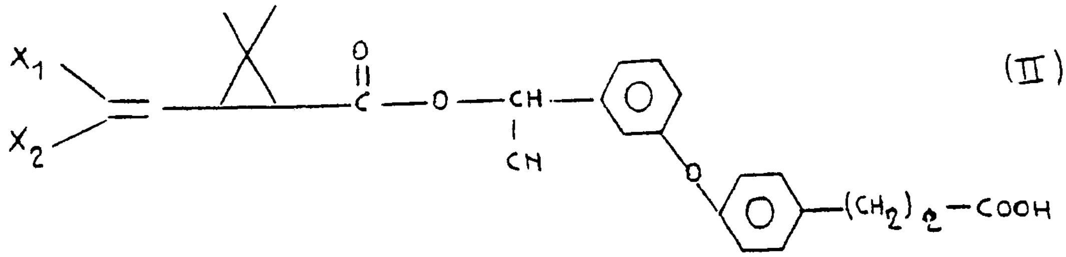

(II)

dans laquelle $X_1$ et $X_2$ ont la signification précitée, en vue de l'activation de la fonction carbonyle, avec un halogénoformiate d'alcoyle, en présence d'une base tertiaire, en milieu anhydre et sous atmosphère inerte, pour obtenir l'anhydride mixte correspondant de formule générale ($III_1$) :

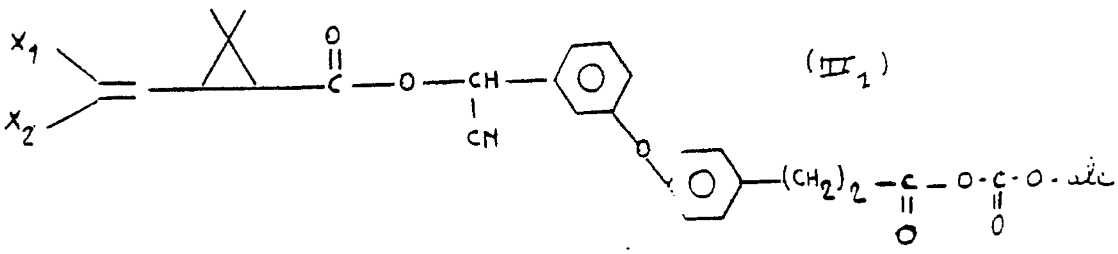

($III_1$)

dans laquelle alc conserve sa signification précédente, que l'on conjugue avec l'albumine serique bovine (B.S.A.) ou avec une autre protéine immunogène et obtient l'antigène cherché.

Dans des conditions plus particulièrement préférées de mise en oeuvre, cette application est caractérisée par les points suivants :
- l'halogénoformiate d'alcoyle est le chloroformiate d'isobutyle et l'on opère en présence de tri-n-butyl amine ;
- on fait réagir l'anhydride mixte de formule générale ($III_1$) avec l'albumine sérique bovine (BSA), ou une autre protéine immunogène préalablement dissoute dans un mélange eau-dioxanne.

L'application décrite permet d'obtenir, au départ du produit de formule (II) :

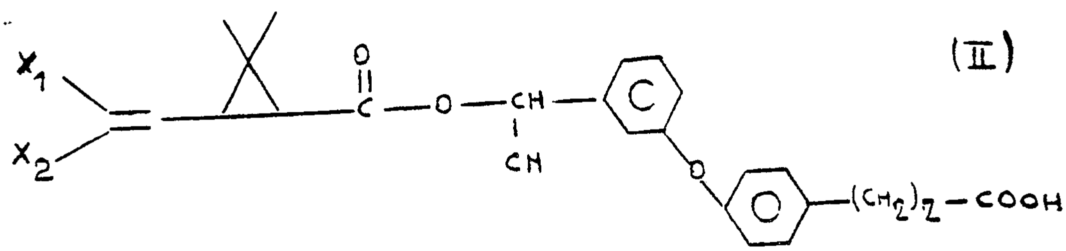

(II)

les antigènes de formule (IV) et (V) :

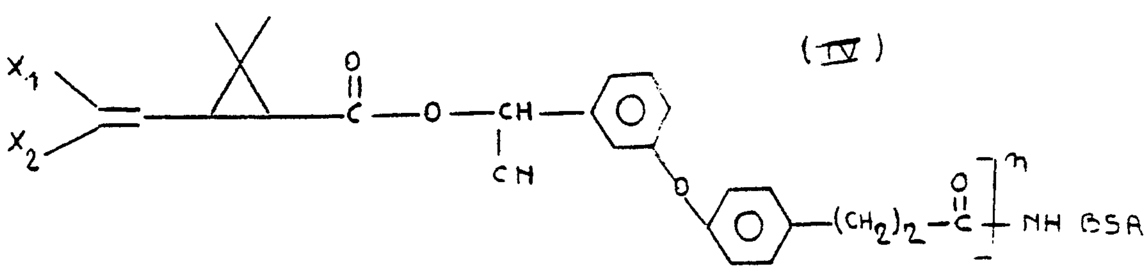

(IV)

dans laquelle n = 10 à 30 et :

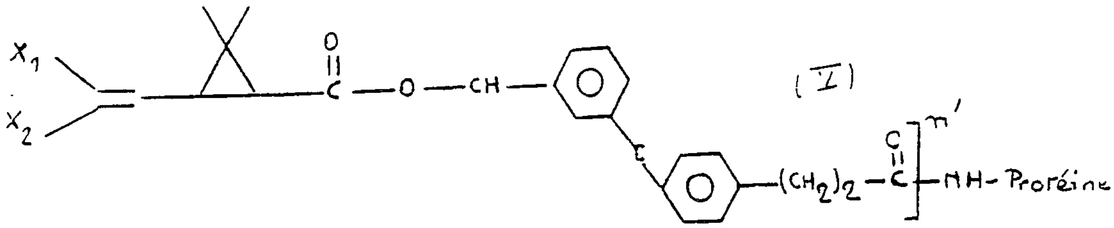

(I)

dans laquelle n' = 10 à 100.

L'invention a également pour objet, à titre de produits industriels nouveaux, les antigènes obtenus lors de l'application décrite ci-dessus, c'est-à-dire les produits de formule générale (IV) et (V).

L'invention a également, pour objet l'application des antigènes ci-dessus à la préparation d'anticorps par injection chez l'animal en présence d'adjuvants.

Les produits de formule générale I, objet de l'invention, peuvent être utilisés lors de études et lors du dosage radioimmunologique des produits B et notamment le produit A pour la deltaméthrine.

Lors de ces études, les produits de formule (I) permettent un dosage spécifique aisé de quantités de l'ordre de quelques dizaines de Picogrammes de produit B, sans que l'on soit obligé de recourir à des méthodes d'isolement et de purification par chromatographie, avant de procéder au dosage proprement dit selon des méthodes radioimmunologiques conventionnelles telles que celles décrites par :

- S.A. BERGSON et R.S. YALOW, HORMONE 4, p. 557 (1964) et
- G.E. ABRAHAM J. of CHEM. ENDOCRINAL METAB., 29, p. 866 (1969).

Les exemples suivants illustrent l'invention, sans toutefois la limiter.

**Exemple 1 : Acide 1R /1 alpha (R), 2 alpha /4-/3/cyano//(2-(2,2-dibromoéthényl) 3,3-diméthylcyclopropyl/ carbonyl) oxy/méthyl/phénoxy/benzène propanoique.**

**Stade A** : 4-/3-(1,3-dioxolan 2-yl) phénoxy/ benzaldéhyde.

On mélange 12,05 g de parahydroxybenzaldéhyde, 30 cm3 de pyridine et 5,5 g de méthylate de sodium, chauffe pour distiller 4 cm3 de pyridine, ajoute 2,5 g de chlorure cuivreux et 38 g de 2-(m-bromophényl) 1,3-dioxolane, chauffe en agitant énergiquement jusqu'à 200¤C (température extérieure), tout en distillant la pyridine, et maintient à 200¤ C (température extérieure) pendant 5 heures. Après refroidissement, on reprend le résidu au chlorure de méthylène, lave par une solution aqueuse N d'acide chlorhydrique puis à l'eau. On sèche les phases organiques, concentre à sec par distillation sous pression réduite, chromatographie le résidu sur silice en éluant par un mélange hexane-acétate d'éthyle (6/4) et obtient 18,28 g de produit attendu.

**Spectre IR (chloroforme) :**

| | |
|---|---|
| 2 830 cm-1 | |
| 2 738 cm-1 | aldéhyde |
| 1 696 cm-1 | C = 0 |

1 598 cm-1
1 580 cm-1 } aromatique
1 500 cm-1

1 240 cm-1 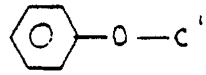

1 097 cm-1 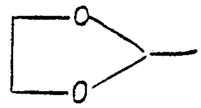
1 071 cm-1
1 076 cm-1

833 cm-1 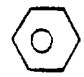

Spectre de RMN ($CDCl_3$)

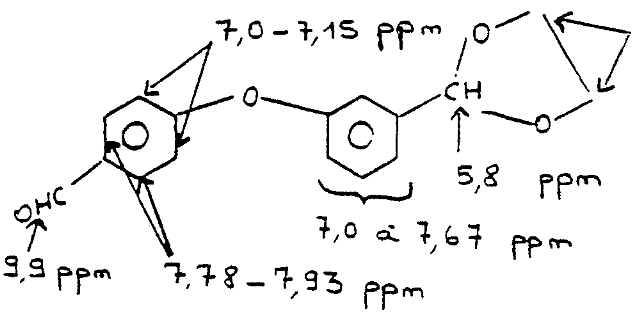


**Stade B** : 2-/3-(4-/delta E (1,1-diméthyléthoxy) 1-oxopropényl/phénoxy) phényl/1,3-dioxolane.

On mélange 137 cm3 de tétrahydrofuranne et 13,72 g de bromure de lithium, refroidit à -30¤C, ajoute d'un coup 11,94 cm3 de diisopropylamine, puis un mélange de 10 g de 4-/3-(1,3-dioxolan 2-yl) phénoxy/ benzaldéhyde, 200 cm3 de tétrahydrofuranne et 10,09 g de 0,0-diéthylterbutyloxycarbonylméthyl phosphonate, agite pendant 16 heures à -15¤c, verse le mélange réactionnel dans l'eau, extrait à l'éther éthylique, concentre à sec par distillation sous pression réduite, chromatographie le résidue sur silice en éluant par un mélange d'hexane et d'acétate d'éthyle (8/2) et obtient 13,48 g de produit attendu.

Spectre IR (chloroforme) :

1 700 cm-1 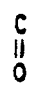

1 634 cm-1 C = C

1 602 $cm^{-1}$
1 590 $cm^{-1}$
1 506 $cm^{-1}$ } aromatique
1 487 $cm^{-1}$

982 $cm^{-1}$ -CH = CH-

Spectre de RMN
$(CD\ Cl_3)$

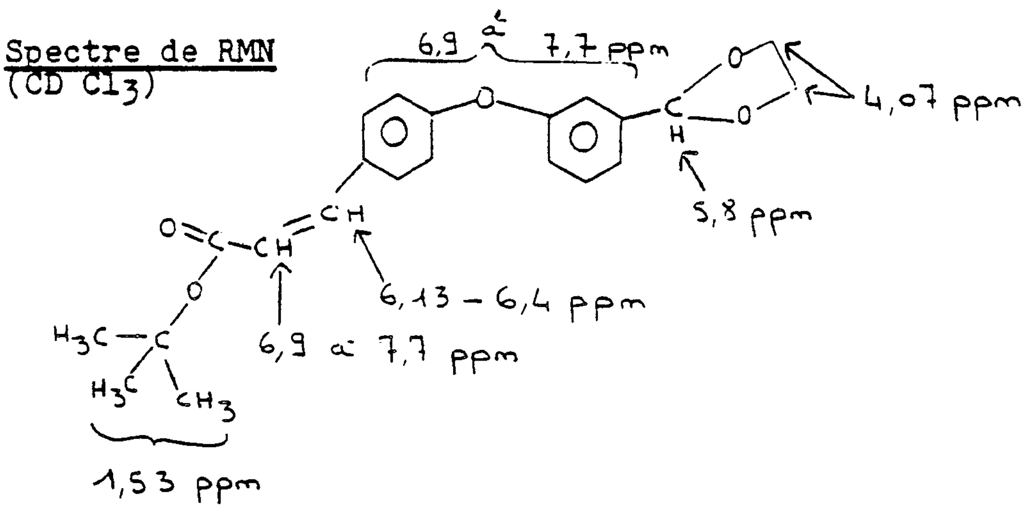


**Stade C** mélange de : diméthoxy méthyl 3-(4-/delta E (1,1-diméthoxy éthoxy) 1-oxopropényl)phénoxy benzène et de méthoxy méthyl 3-(4-/delta E (1,1-diméthoxy éthoxy) 1-oxopropényl/ phénoxy) benzène.

On mélange 1,5 g d'hydroxyde de palladium déposé sur sulfate de baryum à 5% de palladium et 10 cm3 de méthanol, introduit une solution de 12 g de 2-/3-(4-/delta E (1,1-diméthyléthoxy 1-oxopropényl/phénoxy) phényl/ 1,3-dioxolane, agite sous hydrogène pendant 1 heure et 15 minutes en absorbant 717 cm3 d'hydrogène, on filtre, concentre à sec le filtrat par distillation sous pression réduite, chromatographie le résidu sur silice en éluant par un mélange d'hexane et d'acétate d'éthyle (9/1) et isole 7,16 g d'un mélange 50/50 des deux composés :

Spectre IR (chloroforme) :

1 605 $cm^{-1}$
1 586 $cm^{-1}$
1 506 $cm^{-1}$ } aromatique
1 483 $cm^{-1}$

1 250 $cm^{-1}$ 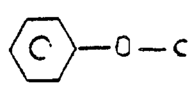

1 720 $cm^{-1}$ C=O de $CO_2$ tBu

1 369 $cm^{-1}$ méthyle

1 147 cm-1 C-O-C

Spectre de RMN ($CDCl_3$) :

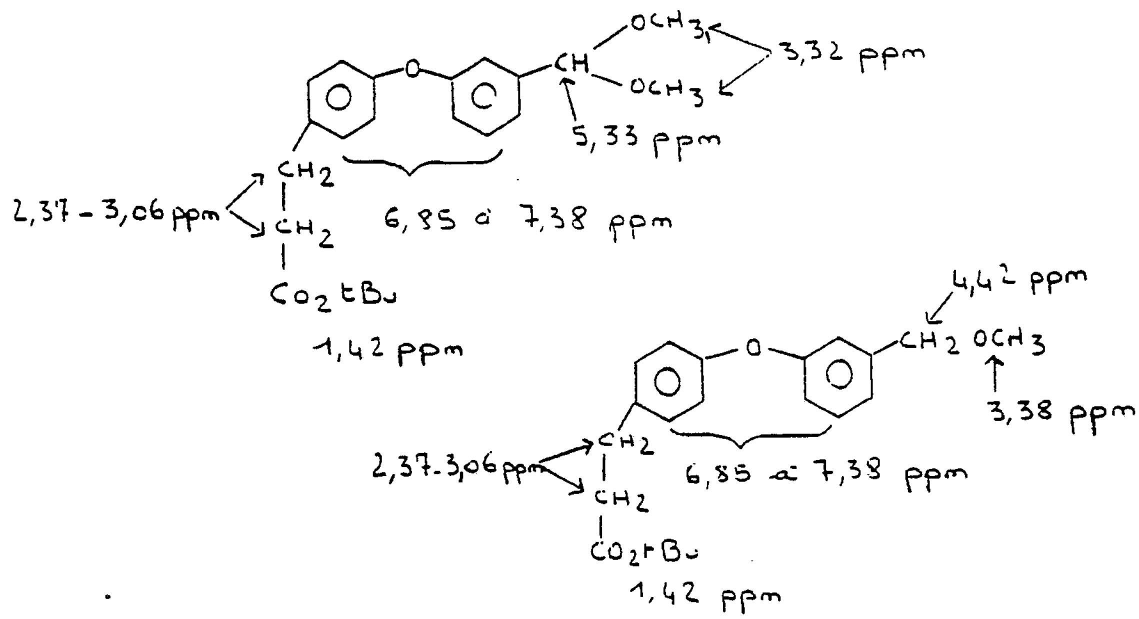


**Stade D** : 3/4/(1,1-diméthyléthoxy) 1-oxopropyl/phénoxy/benzaldéhyde.

On mélange 6 g de mélange de composés obtenu au stade C, 30 cm3 d'acétone et 24 cm3 de solution aqueuse N d'acide chlorhydrique, ajoute une quantité d'acétone nécessaire pour obtenir une seule phase (soit 42 cm3), agite pendant 2 heures et 30 minutes à 20¤C, verse dans l'eau, extrait au chlorure de méthylène, concentre à sec par distillation sous pression réduite, chromatographie le résidu sur silice en éluant par un mélange d'hexane et d'acétate d'éthyle (9/1) et obtient 2,74 g d'aldéhyde attendu.

Spectre 1R (chloroforme) :

2 740 cm-1 } C-H
2 820 cm-1 }

1 701 cm-1 C = O

1 720 cm-1 C = O de $CO_2$ tBu

| Spectre de RMN : | |
|---|---|
| 1,45 | ppm H du ter butyle |
| 2,38 à 3,1 | ppm H de $CH_2$ |
| 6,9 à 7,65 | ppm H aromatiques |
| 10,01 | ppm H de CHO. |

**Stade E** : .1R-(1 alpha (S), 3 alpha) /3-(2,2-dibromoéthényl) 2,2-diméthyl cyclopropane carboxylate de cyano/3-/4-/3-(1,1-diméthyl éthoxy) 3-oxopropyl/ phénoxy/ phényl/ méthyle et /1R-(1 alpha (R), 3 alpha/ 3-(2,2-dibromoéthényl) 2,2-diméthyl cyclopropane carboxylate de cyano/ 3-/4-/3-(1,1-diméthyléthoxy) 3-oxopropyl/phénoxy/phényl/méthyle.

On mélange 0,45 g de cyanure de sodium 0,0137 g de bromure de tétradécyl triméthyl ammonium, 8,22 cm3 d'eau, introduit à 20¤C sous agitation vigoureuse un mélange de 2,74 g de 3-/4-/(1,1-diméthyléthoxy) 1-oxopropyl/ phénoxy/ benzaldéhyde obtenu au stade D, 0,0137 g de bromure de tétradécyl triméthyl ammonium, 2,74 cm3 de toluène et un mélange de 2,68 g de chlorure de l'acide 1R, cis 3-(2,2-dibrométhényl) 2,2-diméthyl cyclopropane carboxylique et de 2,74 cm3 de toluène, agite pendant 3 heures à 20¤C, ajoute une solution aqueuse N d'acide chlorhydrique, jusqu'à pH = 7, décante, lave à l'eau la phase organique, extrait au toluène la phase aqueuse, concentre à sec les solutions toluéniques réunies par distillation sous pression réduite, chromatographie le résidu sur silice en éluant par un mélange d'hexane et d'acétate d'éthyle (9/1) et obtient 4,92 gde mélange brut des isomères (R) et (S).

On chromatographie le mélange brut sur silice en éluant par un mélange d'hexane et d'éther isopropylique (7/3) et obtient 3 fractions.

a/. 1,46 g de fraction contenant l'isomère R,
b/. 2,64 g de fraction contenant un mélange des isomères R et S.
c/. 0,395 g de fraction contenant l'isomère S.

La fraction b) est à son tour purifiée par chromatographie sur silice en éluant par un mélange d'hexane et d'éther isopropylique (7/3) et obtient 2 fractions :

d/ 0,67 g de fraction contenant l'isomère R
e/ 1,66 g de fraction contenant l'isomère S

<u>Spectre IR</u> :

| (chloroforme) | isomère R | isomère S |
|---|---|---|
| 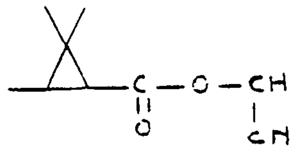 | 1 736 $cm^{-1}$ | 1 736 $cm^{-1}$ |
| 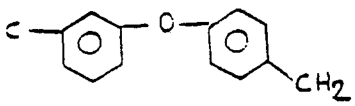 | 1 603 $cm^{-1}$<br>1 591 $cm^{-1}$<br>1 506 $cm^{-1}$ | 1 603 $cm^{-1}$<br>1 591 $cm^{-1}$<br>1 506 $cm^{-1}$ |
| | 1 487 $cm^{-1}$ (max) | 1 487 $cm^{-1}$ (max) |
| $CH_2-CO_2tBu$<br>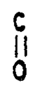 | 1 722 $cm^{-1}$ | 1 722 $cm^{-1}$ |
| Me | 1 370 $cm^{-1}$ | 1 370 $cm^{-1}$ |
| C-O-C | 1 148 $cm^{-1}$ | 1 148 $cm^{-1}$ |

| Spectre RMN (CD $Cl_3$) | | | | |
|---|---|---|---|---|
| | isomère R | | isomère S | |
| hydrogènes des méthyles géminés | 1,31 | ppm | 1,2-1,25 | ppm |
| hydrogène de trBu | 1,44 | ppm | 1,42 | ppm |
| les hydrogènes du cyclopropyle | 1,82 à 2,18 | ppm | 1,75 à 2,2 | ppm |
| les hydrogènes des $CH_2$ de la chaîne alkyle | 2,37 à 3,07 | ppm | 2,33 à 3,0 | ppm |
| hydrogène de -CH-CN | 6,33 | ppm | 6,37 | ppm |
| hydrogènes éthyléniques | 6,65 à 6,7 | ppm | 6,63 à 6,77 | ppm |
| hydrogènes aromatiques | 6,88 à 7,43 | ppm | 6,86 à 7,4 | ppm |

| Dichroïsme circulaire : | | | | | | |
|---|---|---|---|---|---|---|
| | isomère R | | | isomère S | | |
| Max | ≤ 230 nm $\Delta\epsilon$ | = | -9 | max vers 225 mn $\Delta\epsilon$ | = | +11 |
| Max | 268 nm $\Delta\epsilon$ | = | -0,4 | max 285 mn | | |
| Max | 280 nm $\Delta\epsilon$ | = | -0,45 | $\Delta\epsilon$ | = | + 0,4 |

**<u>Stade F</u>** : Acide 1R-/1 alpha (R), 2 alpha/ 4-/3-/cyano/ /3-(2,2-dibromoéthényl) 2,2-diméthyl cyclopropyl/carbonyl/oxy/méthyl/phénoxy/benzène propanoïque.

On mélange 1,1 g d'ester terbutylique de l'isomère R obtenu au stade précédent avec 55 cm3 de

toluène, porte au reflux, ajoute 0,1 g d'acide paratoluène sulfonique, maintient le reflux pendant 30 minutes, refroidit, filtre, concentre à sec par distillation sous pression réduite, chromatographie le résidu sur silice en éluant par un mélange d'hexane et d'acétate d'éthyle (1/1) et obtient 0,93 g d'acide attendu.

Spectre IR (chloroforme) :

1 742 $cm^{-1}$ $C=O$ ester

1 604 $cm^{-1}$
1 591 $cm^{-1}$
1 509 $cm^{-1}$
1 487 $cm^{-1}$ } aromatiques

1 712 $cm^{-1}$ acide $C=O$ dimère

| Spectre RMN ($CD\ Cl_3$) | | |
|---|---|---|
| hydrogènes des méthyles géminés et du tBu | 1,3 | ppm |
| hydrogènes du cyclopropyle (cis) | 1,8 à 2,19 | ppm |
| les hydrogènes des $CH_2$ de la chaîne alkyle | 2,5 à 3,17 | ppm |
| hydrogène de -CH-CN | 6,23 | ppm |
| hydrogène éthylénique | 6,6-6,73 | ppm |
| hydrogènes aromatiques | 6,88 à 7,43 | ppm |

**Exemple 2 : acide 1R-/1 alpha (S), 2 alpha /4-/3-/cyano///3-(2,2-dibromo éthényl) 2,2-diméthyl cyclopropyl/ carbonyl/ oxy/ méthyl/ phénoxy/ benzène propanoïque.**

On mélange 1,805 g d'ester terbutylique (S) obtenu à l'exemple 1 avec 90,25 cm3 de toluène, porte au reflux, ajoute 0,18 g d'acide paratoluène sulfonique, maintient le reflux pendant 30 minutes, refroidit, filtre, concentre à sec par distillation sous pression réduite, chromatographie le résidu sur silice en éluant par un mélange d'hexane et d'acétate d'éthyle (1/1) et obtient 1,33 g d'acide attendu.

Spectre IR (chloroforme) :

1 742 $cm^{-1}$ $C=O$ ester

1 604 $cm^{-1}$
1 591 $cm^{-1}$
1 509 $cm^{-1}$
1 487 $cm^{-1}$ } aromatiques

1 712 $cm^{-1}$ acide $C=O$ dimère

| Spectre RMN (CO $Cl_3$) | | |
|---|---|---|
| les hydrogènes des méthyles géminés : | 1,2-1,25 | ppm |
| les hydrogènes du cyclopropyle : | 1,82 à 2,23 | ppm |
| les hydrogènes des $CH_2$ de la chaîne alkyle | 2,5 à 3,17 | ppm |
| hydrogène de -CH-CN | 6,43 | ppm |
| hydrogène éthylénique | 6,68 à 7,16 | ppm |
| hydrogènes aromatiques | 6,97 à 7,7 | ppm |

**Exemple 3 : /1R /1 alpha (S), 2 alpha /4-/3-/cyano///3-(2,2-dibromo éthényl) 2,2-diméthyl cyclopropyl/ carbonyl/ oxy/ méthyl/ phénoxy/benzène N-/2-(2-iodo$^{125}$I 4-1H imidazolyl) éthyl/ propanamide.**

**Stade A**: Préparation de l'anhydride mixte en solution (solution A).

On mélange sous argon 25 µl de dioxane anhydre, 10 µl de tri-n-butylamine dilué au 1/125 dans du dioxane anhydre et 10 µl de chloroformiate d'isobutyl dilué au 1/250 dans du dioxane anhydre, ajoute une solution de 150 g d'acide 1R /1 alpha (S), 2 alpha/4-/3-/cyano///3-(2,2-dibromoéthényl) 3,3-diméthyl cyclopropyl/ carbonyl/ oxy/méthyl/phénoxy/benzène propanoïque en solution dans 25 µl de dioxane anhydre, maintient 30 minutes à 20¤C sous agitation, dilue le mélange réactionnel par 125 µl de dioxane anhydre et obtient la solution A d'anhydride mixte.

**Stade B** : Marquage de l'histamine

On mélange à 20¤c 10 µl de solution d'histamine base 2 mM dans du tampon au phosphate de sodium 0,5 M à pH 7,4, 1 m Ci de $^{125}$ INa, 10 µl de solution aqueuse de chloramine T à 5 mg/ml, après 90 secondes, on ajoute 10 µl de solution aqueuse de métabisulfite de sodium à 25 mg/ml.

Le milieu obtenu B est contrôlé par chromatographie. (voir tableau A).

**Stade C** : Condensation :

Dans le tube contenant le milieu réactionnel B, on introduit 50 I de solution A d'anhydride mixte, conserve pendant 2 heures à 4¤C. On obtient alors la solution C qui est analysée par chromatographie (voir tableau B).

**Stade D** : Purification.

Le milieu réactionnel C est purifié par HPLC sur une colonne Zorbax Golden Sil 100 x 7,5 nm, 3 m, élué par le mélange chloroforme-méthanol (92/8). La radioactivité de l'effluent est suivie grâce à un détecteur à cristal d'iodure de sodium Beckman 170 couplé à un enregistreur intégrateur. On obtient 4 pics (voir tableau I). Seul le pic 3 contient un antigène qui se lie à un anticorps "antideltaméthrine". Les fractions contenant le produit immunoréactif sont réunies. La solution D (tableau C) obtenue est concentrée à sec sous argon, le résidu est additionné de 5 ml d'éthanol. On obtient environ 600 µ Ci de conjugué radioactif. Le produit stocké à -30¤ C est stable pendant au moins 2 mois.

TABLEAU 1

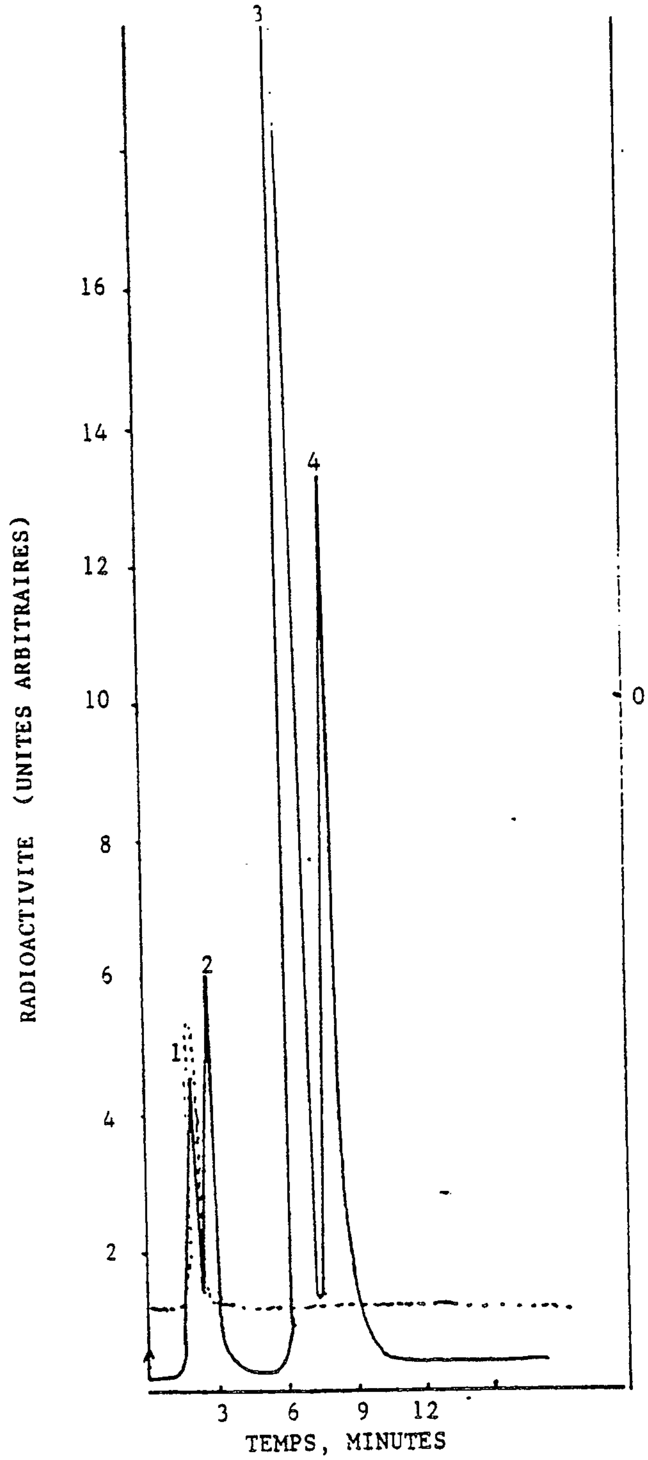


Détection de la Radioactivité
Beckman model 170

| N°des pics | tps de rétention en | % de radio-activité |
|---|---|---|
| 1 | 2,44 | 5,34 |
| 2 | 3,17 | 8,00 |
| 3 | 6,93 | 63,46 |
| 4 | 8,57 | 22,98 |

Profil HPLC du conjugué iodé. Conditions de chromatographie : colonne : Zorbax Golden Sil, 3 µm ; phase mobile : $CHCl_3$'MeOH (98/2,v/v) ; débit : lml/min ; Pression : 800 PSI ; Echantillon 25 µl ; détection : Radioactivité - Beckman Model 170, UV ... UVICORD LKB 276 nm 0,02.

TABLEAU A

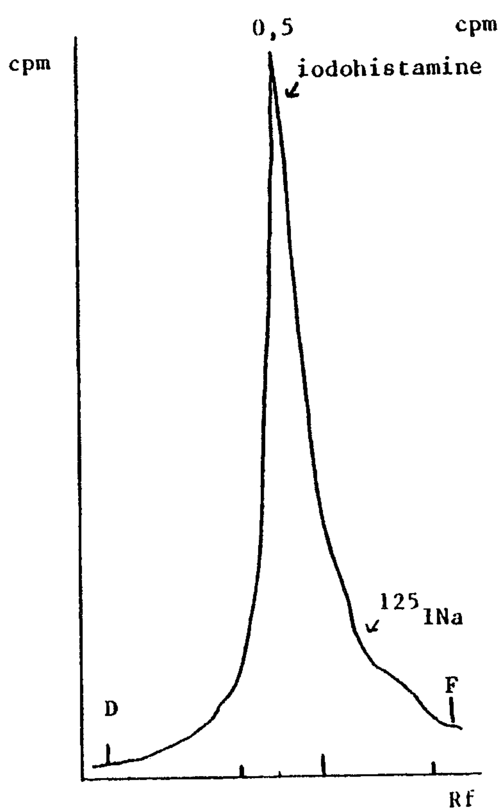


TABLEAU B

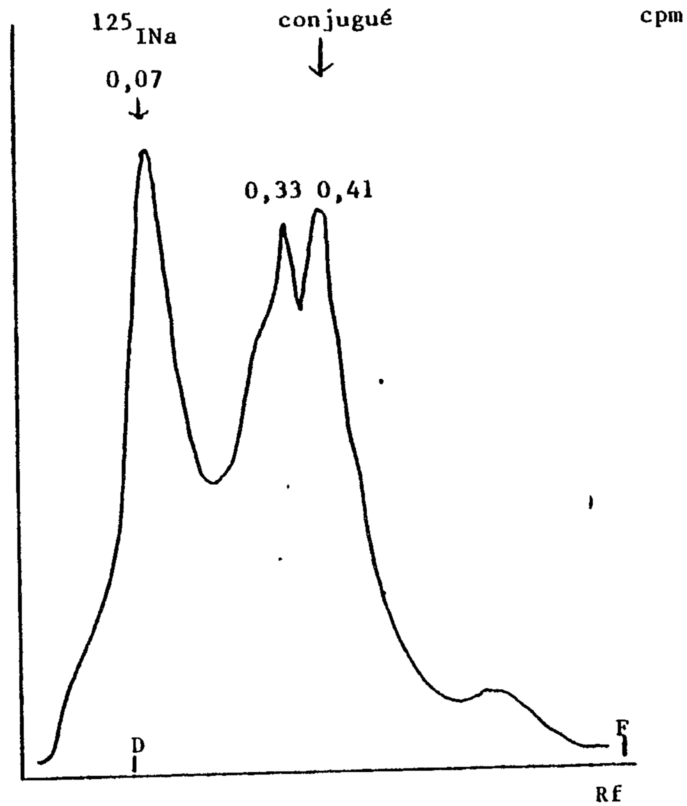


TABLEAU C

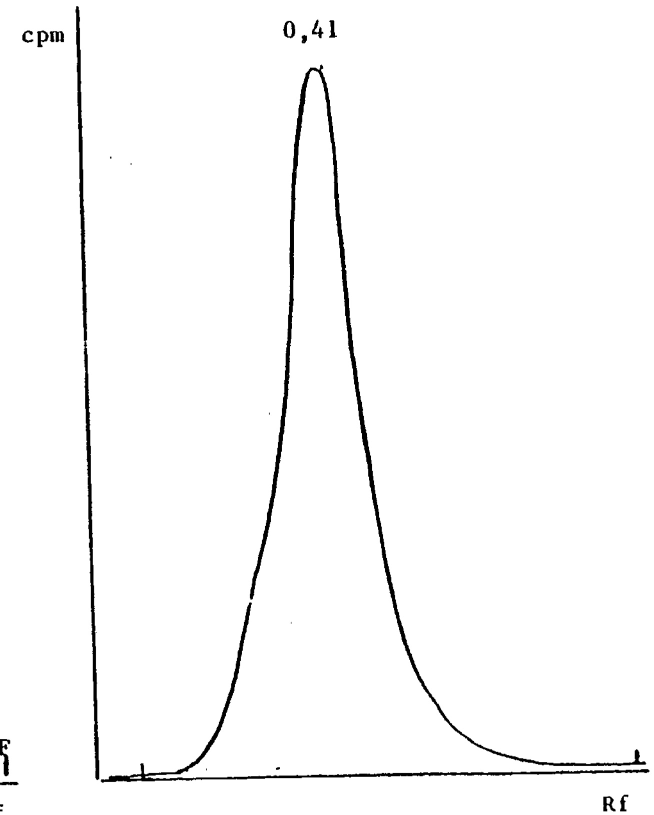


Radiochromatogrammes de A : [125]Iodohistamine (milieu "B") ; B : conjugué radioactif (milieu "C") ; C pic 3 conjugué purifié (solution "D") ; Plaques de Silice Merck 60F254 ; Solvants A : $EtOH-H_2O-NH_4OH$ (77/18/5) ; B et C : cyclohexane - EtOH - Triéthylamine (70/30/1). Lecteur de plaques : Berthold LB 2832.

**Exemple 4 : /1R /1 alpha (R), 2 alpha /4-/3-/cyano///3-(2,2-dibromo éthényl) 2,2-diméthyl cyclopropyl/carbonyl/oxy/méthyl/phénoxy/benzène N-/2-(2-iodo $^{125}$ I 4-1H-imidazolyl) éthyl/ propanamide.**

Stade A: Préparation de l'anhydride mixte en solution (solution A).

On mélange, sous argon, 25 µl de dioxane anhydre, 10 µl de tri-n-butyl amine dilué au 1/125 dans du dioxane anhydre, 10 µl de chloroformiate d'isobutyl dilué au 1/250 dans du dioxane anhydre, ajoute 150 µ g d'acide 1 R /1 alpha (R), 2 alpha/ 4-/3-/cyano///3-(2,2-dibromoéthényl) 2,2-diméthyl cyclopropyl/ carbonyl/ oxy/ méthyl/ phénoxy/benzène propanoïque en solution dans 25 µl de dioxane anhydre, conserve pendant 30 minutes à 20¤C, dilue le mélange réactionnel par 125 µl de dioxane anhydre et obtient la solution A d'anhydride mixte.

Stade B : Marquage de l'histamine

On mélange à 20¤C 10 µl de solution d'histamine base 2 mM dans du tampon phosphate de sodium 0,5 M à pH 7,4, 1 m Ci de $^{125}$ I Na, 10 µl de solution aqueuse de chloramine T à 5 mg/ml, après 90 secondes, on ajoute 10 µl de solution aqueuse de métabisulfite de sodium à 25 mg/ml.

Le milieu obtenu B est contrôlé par chromatographie (voir tableau A).

Stade C : Condensation

Dans le tube contenant le milieu réactionnel B, on introduit 50 l de solution A d'anhydride mixte, conserve pendant 2 heures à 4¤C. On obtient alors la solution C qui est analysée par chromatographie (voir tableau B).

Stade D : Purification.

Le milieu réactionnel C est purifié par HPLC sur une colonne Zorbax Golden Sil 100 x 7,5 nm, 3 µ m, éluée par le mélange chloroforme-méthanol (97/3). La radioactivité de l'effluent est suivie grâce à un détecteur à cristal d'iodure de sodium Berthold. On obtient 4 pics (voir tableau I). Seul le pic 3 contient un antigène capable de se lier aux anticorps "antideltaméthrine". Les fractions contenant le produit immunoréactif sont réunies. La solution D (tableau C) obtenue est évaporée à sec sous argon. Le résidu est additionné de 5 ml d'éthanol. On obtient 220 µ Ci de conjugué radioactif. Le produit stocké à -30¤ C est stable pendant au moins 2 mois.

TABLEAU 1

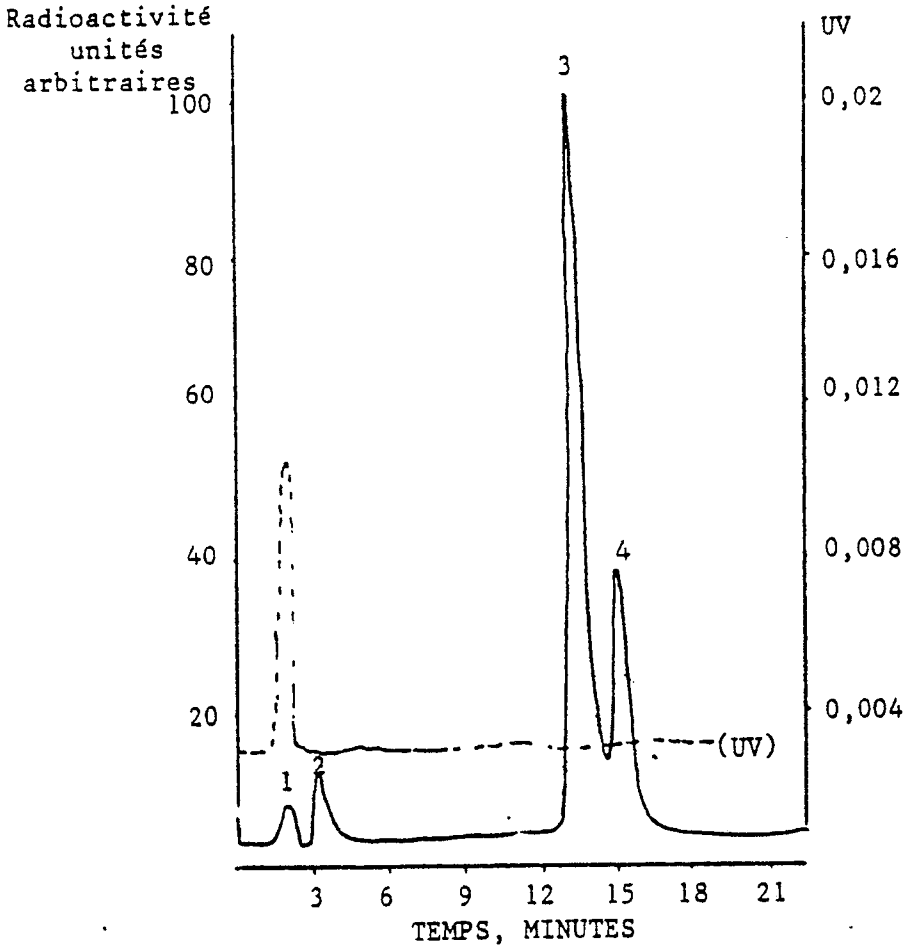


Profil HPLC du conjugué iodé - Conditions de chromatographie : Colonne : Zorbax Golden Sil, 3 um ; Phase mobile : $CHCl_3$/MeOH 97/3 (v/v) ; débit : 1 ml/min ; Pression : 800 PSI ; Echantillon 70 µl, détection : radioactivité Berthold SOOK, UV. UVICORDS LKB 276 nm 0,02.

TABLEAU A

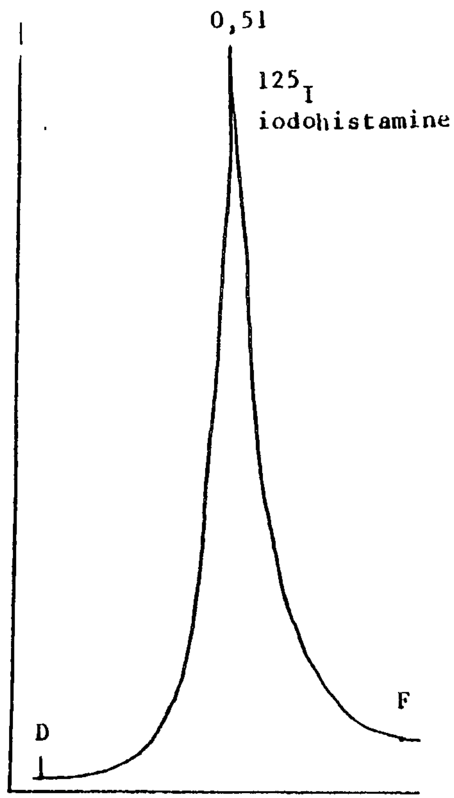


TABLEAU B

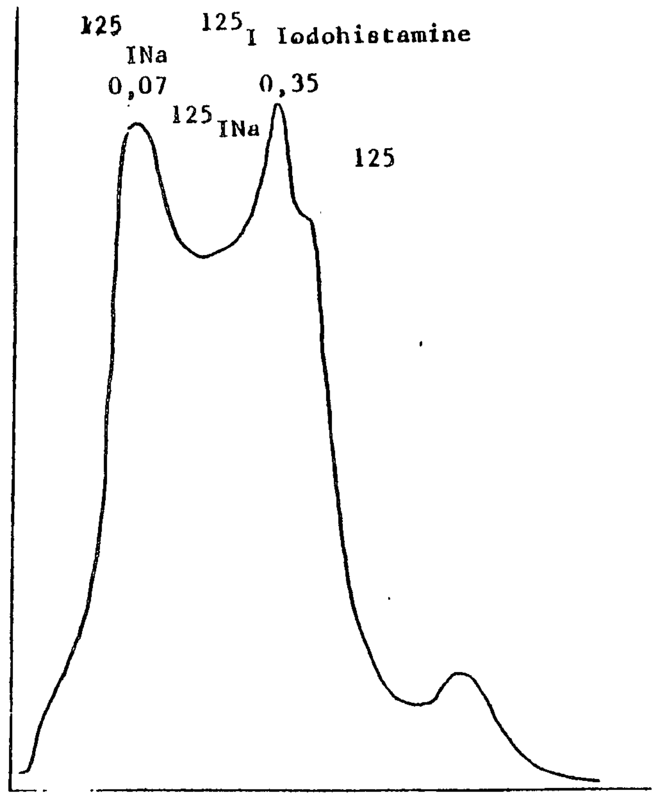


TABLEAU C

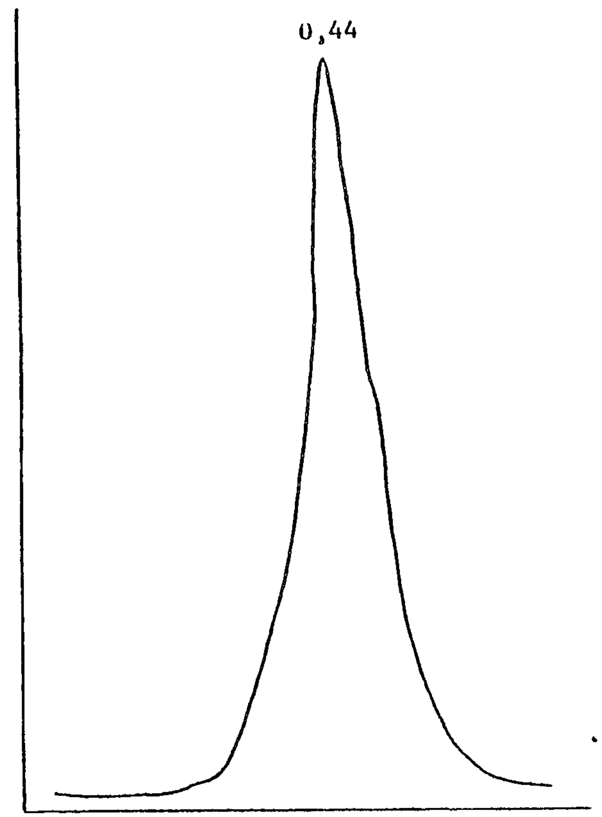


Radiochromatogrammes de A : $^{125}$Iodohistamine (milieu "B"), B : conjugué radioactif (milieu "C"), C : pic 3 (solution D), Plaques de Silice Merck 60F254, Solvants A : EtOH-$H_2O$-$NH_4OH$ (77/18/5), B et C : Cyclohexane-EtOH-Triéthylamine (70/30/1), Lecteur de plaques Berthold LB 2832.

**Exemple 5 : Conjugué d'albumine serique bovine et d'acide 1R /1 alpha (S), 2 alpha/ 4-/3-/cyano///3-(2,2-dibromo éthényl) 2,2-diméthyl cyclopropyl/ carbonyl/ oxy/ méthyl/ phénoxy/ benzène propanoïque.**

**Stade A** : Préparation de l'anhydride mixte.

On mélange 115,5 mg d'acide 1R/ 1 alpha (S), 2 alpha /4-/3-/cyano/// 3-(2,2-dibromoéthényl) 2,2-diméthyl cyclopropyl/carbonyl/oxy/méthyl/ phénoxy/benzène propanoïque, 2 ml de dioxane anhydre, ajoute un mélange de 47 µl de tri-n-butyl amine et de 27 µl de chloroformiate d'isobutyle, agite pendant 30 minutes à 13¤C.

**Stade B** : Préparation de la solution de BSA.

On mélange 8 ml d'eau, 276 mg de BSA, 800 µl de dioxane, agite pendant 20 minutes à +4¤C, ajoute 18 ml de dioxane, note la formation d'un précipité blanc laiteux qui disparait par addition de 100 µl de solution aqueuse N de soude, ajuste le pH de la solution à 7,9-8,0.

**Stade C** : Condensation.

A la solution de BSA, on ajoute lentement à +4¤C la solution d'anhydride mixte, note la formation d'un précipité laiteux. Le pH passe de 8,0 à 7,4, valeur à laquelle il est maintenu durant 5 heures d'agitation à +4¤C.

**Stade D** : Purification.

La solution laiteuse obtenue est mise à dialyser dans un boyau Visking 36/32 de 20 x 3 cm, contre 6 fois 5 litres d'eau.

Le résidue obtenu est lyophilisé durant 48 heures dans un appareil Usifroid SMH 015. On obtient 311 mg d'un solide blanc.

| Analyse : | | |
|---|---|---|
| % | Br | 8,7 |
| % | N | 11,9 |

Spectre UV, dichroïsme circulaire : chromophore

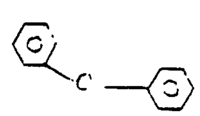

absorbant dans la même région que la BSA, ce qui rend le dosage imprécis. Estimation : $6.10^{-5}$ mole/g de produit.

**Exemple 6 : conjugué d'albumine serique bovine et d'acide 1R /1 alpha (R), 2 alpha/ 4-/3-/cyano///3-(2,2-dibromo éthényl) 2,2-diméthyl cyclopropyl/ carbonyl/ oxy/ méthyl/ phénoxy/ benzène propanoïque.**

Stade A : Préparation de l'anhydride mixte.

On mélange 115,5 mg d'acide 1R/1 alpha (R), 2 alpha /4-/3-/cyano/// 3-(2,2-dibromoéthényl) 2,2-diméthyl cyclopropyl/ carbonyl/ oxy/ méthyl/ phénoxy/ benzène propanoïque, 2 ml de dioxane anhydre, ajoute un mélange de 47 µl de tri-n-butyl amine et de 27 µl de chloroformiate d'isobutyle, agite pendant 30 minutes à 15¤C.

Stade B : Préparation de la solution de BSA.

On mélange 8 ml d'eau, 276 mg de BSA, 800 µl de dioxane, agite pendant 20 minutes à +4¤C, ajoute 18 ml de dioxane, note la formation d'un précipité blanc laiteux qui disparait par addition de 50 µl de solution aqueuse N de soude, ajoute le pH de la solution à 7,9-8,0.

Stade C : Condensation.

A la solution de BSA, on ajoute lentement à +4¤C la solution d'anhydride mixte, il y a formation d'un précipité laiteux. Le pH passe de 8,0 à 7,5, valeur à laquelle il est maintenu durant toute l'agitation (5 heures à +4¤C).

Stade D : Purification.

La solution laiteuse obtenue est mise à dialyser dans un boyau Visking 36/32 de 3 x 15 cm, contre 12 fois 5 litres d'eau.

On obtient une suspension qui est lyophilisée durant 48 heures dans un appareil Usifroid SMH 015.

On obtient 329 mg d'un solide blanc qui sera utilisé pour produire des anticorps par injection chez l'animal.

| Analyse : | | |
|---|---|---|
| % | Br | 9,5 |
| % | N | 11,5 |

**Revendications**

1) Application des produits de formule (II) :

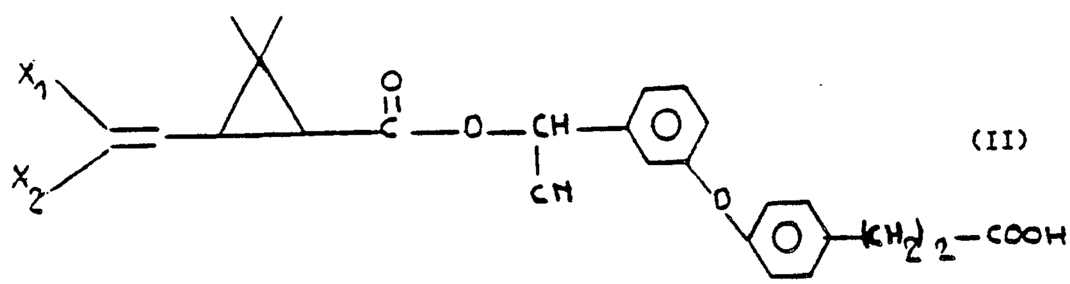

(II)

dans laquelle $X_1$ représente un atome d'halogène ou un radical trifluorométhyl, $X_2$ représente un atome d'halogène à la préparation d'antigènes, caractérisée en ce que l'on conjugue ces produits ou une forme activée de ces produits avec de l'albumine sérique bovine (B.S.A.) ou avec une autre protéine immunogène et obtient les antigènes cherchés.

2) Application selon la revendication 1, caractérisée en ce que $X_1$ représente un radical de chlore ou de brome ou un radical trifluorométhyle et $X_2$ représente un atome de chlore ou de brome.

3) Application selon la revendication 2 caractérisée en ce que $X_1$ et $X_2$ représentent un atome de brome.

4) Application selon l'une quelconque des revendications 1 à 3 caractérisée en ce que l'on fait réagir un produit de formule (II) dans laquelle $X_1$ et $X_2$ ont la signification précitée, en vue de l'activation de la fonction carbonyle, avec un halogénoformiate d'alcoyle, en présence d'une base tertiaire, en milieu anhydre et sous atmosphère inerte, pour obtenir l'anhydride mixte correspondant de formule générale ($III_1$) :

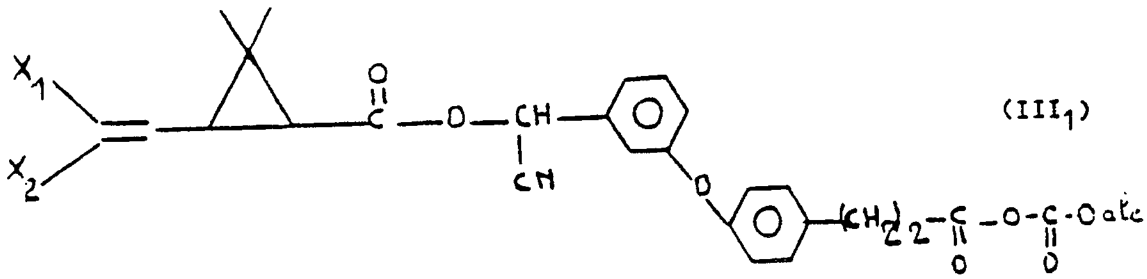

$(III_1)$

dans laquelle alc a la signification indiquée à la revendication 8 et $X_1$ et $X_2$ ont la signification précitée, que l'on conjugue avec l'albumine serique bovine (B.S.A.) ou une autre protéine immunogène et obtient l'antigène cherché.

5) Application selon la revendication 4, caractérisée en ce que :

- l'halogénoformiate d'alcoyle est le chloroformiate d'isobutyle et l'on opère en présence de tri-n-butyl amine ;
- on fait réagir l'anhydride mixte de formule générale (III) avec l'albumine sérique bovine (B.S.A.), ou une autre protéine immunogène préalablement dissoute dans un mélange eau-dioxane.

6) Antigènes constituées par la conjugaison du produit de formule (II), selon la revendication 1 avec de l'albumine serique bovine (B.S.A.) ou avec une autre protéine immunogène, à savoir les antigènes de formule (IV) et (V) :

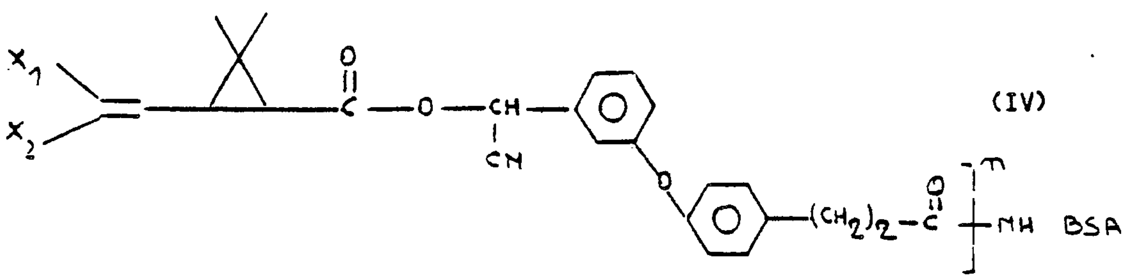

(IV)

dans laquelle n = 10 à 30 et :

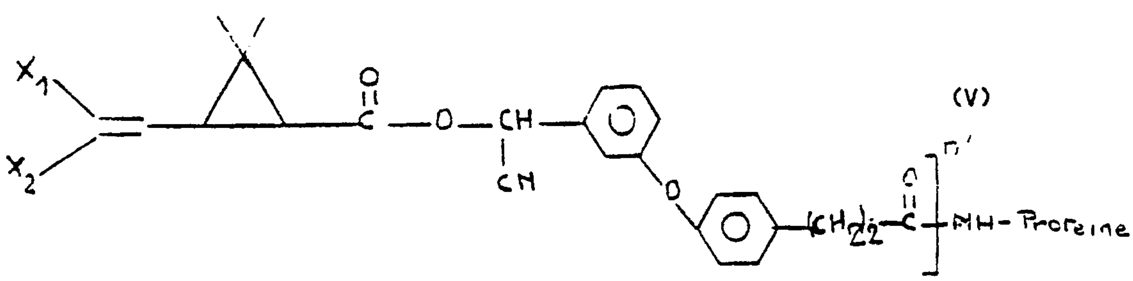

(V)

dans laquelle n′ = 10 à 100.

7) Application des antigènes selon la revendication 6, à la préparation d'anticorps.

Office européen des brevets

# RAPPORT DE RECHERCHE EUROPEENNE

Numero de la demande

EP 89 11 2013

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 136 159 (M.J. STONE)<br>* En entier *<br>----- | 1-7 | G 01 N 33/534//<br>C 07 C 121/75<br>C 07 B 59/00<br>C 07 D 233/68 |
| | | | **DOMAINES TECHNIQUES RECHERCHES (Int. Cl.4)** |
| | | | G 01 N 33/00<br>C 07 C 121/00<br>C 07 B 59/00<br>C 07 D 233/00 |

Le présent rapport a été établi pour toutes les revendications

| Lieu de la recherche | Date d'achèvement de la recherche | Examinateur |
|---|---|---|
| LA HAYE | 08-08-1989 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)